# EUROPEAN PATENT APPLICATION

(11) **EP 3 336 125 A1**
(43) Date of publication of application: **20.06.2018**
(21) Application number: 17204244.2
(22) Date of filing: 28.11.2017
(51) Int. Cl.: C08G 61/10, C08G 61/12, C09D 165/02, C08L 65/02, H01L 23/532

(54) **POLYARYLENE RESIN COMPOSITIONS**

(30) Priority: 14.12.2016 US 201662434063 P
(71) Applicant: Rohm and Haas Electronic Materials LLC, Marlborough, MA 01752 (US)
(72) Inventor: GILMORE, Christopher, Marlborough, MA 01752 (US); RACHFORD, Aaron A., Marlborough, MA 01752 (US); ZHANG, Jieqian, Marlborough, MA 01752 (US); DING, Ping, Marlborough, MA 01752 (US); LEE, Dong-Eun, Marlborough, MA 01752 (US); LI, Anton, Marlborough, MA 01752 (US); KIM, Young-Seok, Marlborough, MA 01752 (US)
(74) Representative: Houghton, Mark Phillip

(57) **Abstract**

Polyarylene oligomer compositions capable of curing at lower temperatures than conventional polyarylene oligomers are useful in forming dielectric material layers in electronics applications.

## Description

The present invention relates generally to the field of polyarylene resins and more particularly to the field of polyarylene resin compositions for use in the manufacture of electronic devices.

Polymer dielectrics may be used as insulating layers in various electronic devices, such as integrated circuits, multichip modules, laminated circuit boards, displays and the like. The electronics fabrication industry has different requirements for dielectric materials, such as dielectric constant, coefficient of thermal expansion, modulus, and the like, depending upon the particular application.

Various inorganic materials, such as silica, silicon nitride and alumina, have been used as dielectric materials in electronic devices. These inorganic materials generally can be deposited in thin layers, typically by vapor deposition techniques, and have advantageous properties, such as not readily absorbing water. Polymer dielectric materials often possess properties which offer advantages over inorganic dielectric materials in certain applications, such as ease of application such as by spin-coating techniques, gap-filling ability, lower dielectric constants, and the ability to withstand certain stresses without fracturing, that is, polymer dielectrics can be less brittle than inorganic dielectric materials. However, polymer dielectrics often present challenges to process integration during fabrication. For example, to replace silicon dioxide as a dielectric in certain applications such as integrated circuits, the polymer dielectric must be able to withstand processing temperatures during metallization and annealing steps of the process. In general, the polymer dielectric material should have a glass transition temperature greater than the processing temperature of subsequent manufacturing steps. Also, the polymer dielectric should not absorb water which may cause an increase in the dielectric constant and potential corrosion of metal conductors.

Polyarylene polymers are well-known as dielectric materials and possess many desirable properties. One class of polyphenylene polymers is polyphenylene ethers prepared by oxidative coupling of at least one hydroxyaromatic compound, such as a phenol. Another class of polyphenylene polymers is prepared by a Diels-Alder reaction of certain ethynyl-substituted aromatic compounds and biscyclopentadienone monomers. Polyphenylene polymers prepared by each method have different polymer architectures. The reaction conditions used in oxidative coupling limits the possible substituents on the hydroxyaromatic compounds, such that post-polymerization functionalization is often required if certain substituents are desired that would be sensitive to the oxidation conditions employed.

U.S. Pat. No. 5,017,650 (Nakamura et al.) discloses resin compositions containing: (A) an aromatic polyester; (B) a polyphenylene ether having a repeat unit of 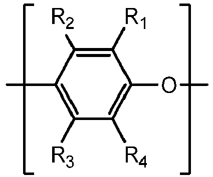 and/or a modified polyphenylene ether obtained by reacting a polyphenylene ether with an active functional group containing monomer having both (a) an aliphatic carbon-carbon double or triple bond and (b) one or more functional groups chosen from radicals of carboxylic, acid anhydride, amide, imide, ester, epoxy, amino, hydroxyl and isocyanate; (C) an epoxy compound of a certain formula; and (D) an impact modifier. None of R₁-R₄ in the above repeating unit are epoxy-reactive moieties. In fact, this patent teaches that the epoxy compound is highly reactive with the aromatic polyester, and the functionalized polyphenylene enhances compatibilization with the epoxy compound. The polyphenylene ethers of U.S. Pat. No. 5,017,650 are not prepared by a Diels-Alder reaction and are functionalized post-polymerization.

International Pat. App. No. WO 87/07286 discloses certain epoxide-functionalized polyphenylene ethers useful in the preparation of polyphenylene ether copolymers. The polyphenylene ethers in this patent are not prepared by a Diels-Alder reaction and are instead prepared by oxidative coupling of at least one monohydroxyaromatic compound. The epoxide-functionalized polyphenylene ethers comprise a plurality of repeat units of the formula 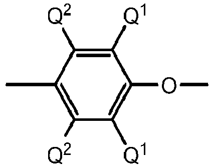 and contain at least one epoxide moiety having the formula 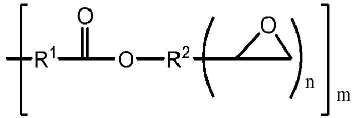 wherein R¹ is a divalent bridging radical containing at least one hydrocarbon group, R² is a polyvalent bridging radical containing at least one hydrocarbon group, m is from 1 to about 5 and n is from 1 to about 10. These polyphenylene ethers are epoxide-functionalized post polymerization, and contain one or two such epoxide moieties bound to the terminal oxygens of the polyphenylene, or contain from 1-5 such epoxide moieties bound to aromatic groups per polyphenylene molecule. Such epoxide-functionalized polyphenylene ethers are useful in the preparation of polyphenylene ether copolymers, which in turn are useful for the compatibilization of polyphenylene ethers with other polymers such as polyesters and polyamides.

International Pat. App. No. WO 97/10193 discloses polyarylene oligomers prepared from certain ethynyl-substituted aromatic compounds and biscyclopentadienone monomers using a Diels-Alder reaction. Films of such polyarylene oligomers typically require heating at a temperature of ≥ 300 °C for a period of time to cure the film. Such high curing temperatures have limited the use of these types of polyarylene oligomers in electronic applications. There remains a need for polyarylene oligomer compositions formed from alkynyl-substituted aromatic compounds and biscyclopentadienone monomers having relatively low dielectric constants and that can be cured at relatively lower temperatures.

The inventors have found that Diels-Alder polymerization conditions used to prepare the present polyarylenes do not present the same substituent limitations that are found in other methods of making polyphenylenes such as in oxidative coupling reactions. The inventors have also found that certain polyarylene polymers having epoxy-reactive moieties can be prepared using Diels-Alder polymerizations. The inventors have further found that such epoxy-reactive moiety-containing polyarylene polymers can be cured at relatively lower temperatures under certain conditions as compared to conventional polyphenylenes prepared using Diels-Alder polymerizations.

The present invention provides a composition comprising: one or more polyarylene polymers having a backbone comprising as repeating units one or more aryl moieties having one or more epoxy-reactive moieties, the polyarylene polymers comprising as polymerized units one or more polyalkynyl-substituted aryl first monomers and one or more biscyclopentadienone second monomers; and one or more epoxide-containing crosslinkers. Also, the present invention provides a method comprising: providing a substrate; coating a layer of the composition of described above on a surface of the substrate; and curing the layer of the composition to form a cross-linked polyarylene layer. The epoxy-reactive moieties are pendant from the polyarylene backbone. As used herein, the term "backbone" refers to the main polymer chain.

The present invention further provides a composition comprising: one or more polyarylene polymers comprising as polymerized units one or more monomers of the formula (1) 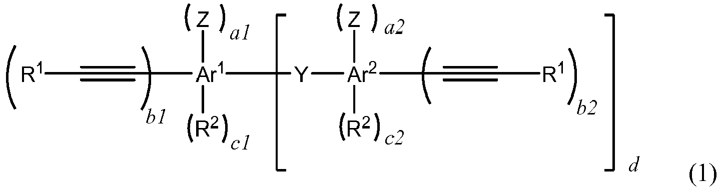 wherein Ar¹ and each Ar² are independently a C₅₋₃₀-aryl moiety; each R¹ is independently chosen from H, C₅₋₃₀-aryl, and substituted C₅₋₃₀ aryl; each R² is independently chosen from C₁₋₁₀-alkyl, C₁₋₁₀-haloalkyl, C₁₋₁₀-alkoxy, CN, and halo; each Z is an epoxy-reactive moiety; Y is a chemical bond or a divalent linking group chosen from -O-, -S-, -S(=O)-, -S(=O)₂-, - C(=O)-, -(C(R⁵)₂)*_{z}*-, C₅₋₃₀-aryl, and -(C(R⁵)₂)*_{z1}*-(C₅₋₃₀ aryl)-(C(R⁵)₂)*_{z2}*-; each R⁵ is independently chosen from H, hydroxy, halo, C₁₋₁₀-alkyl, C₁₋₁₀-haloalkyl, and C₅₋₃₀-aryl; *a1* = 0 to 3; each *a2* = 0 to 3; *b1 =* 1 to 4; each *b2 =* 0 to 2; *c1* = 0 to 2; each *c*2 = 0 to 2; *a1* + each *a2* = 1 to 6; *b1* + each *b2* = 2 to 6; *c1* + each *c2* = 0 to 6; *d* = 0 to 2; *z* = 1 to 10; *z1* = 0 to 10; *z2* = 0 to 10; and *z1* + *z2* = 1 to 10; and one or more epoxide-containing crosslinkers. Still further, the present invention provides a method comprising: providing a substrate; coating a layer of the composition of described above on a surface of the substrate; and curing the layer of the composition to form a cross-linked polyarylene layer.

As used throughout this specification, the following abbreviations shall have the following meanings, unless the context clearly indicates otherwise: °C = degree Celsius; g = gram; mg = milligram; L = liter; mL = milliliter; sec. = second; min. = minute; hr. = hour; DI = deionized; and Da = dalton. Unless otherwise specified, all amounts are percent by weight ("wt%") and all ratios are molar ratios. All numerical ranges are inclusive and combinable in any order, except where it is clear that such numerical ranges are constrained to add up to 100%. The articles "a", "an" and "the" refer to the singular and the plural. "Alkyl" refers to linear, branched and cyclic alkyl unless otherwise specified. "Alkyl" refers to an alkane radical, and includes alkane monoradicals, diradicals (alkylene), and higher-radicals. Unless otherwise noted, "alkyl" includes "heteroalkyl". The term "heteroalkyl" refers to an alkyl group with one or more heteroatoms, such as nitrogen, oxygen, sulfur, or combinations thereof, replacing one or more carbon atoms within the radical, for example, as in an ether or a thioether. In one preferred embodiment, "alkyl" does not include "heteroalkyl". If no number of carbons is indicated for any alkyl or heteroalkyl, then 1-12 carbons are contemplated. "Halo" refers to fluoro, chloro, bromo, and iodo. When an element is referred to as being "disposed on" another element, it can be directly on the other element or intervening elements may be present therebetween. In contrast, when an element is referred to as being "disposed directly on" another element, there are no intervening elements present.

The terms "aromatic moiety" and "aryl" are used interchangeably. As used herein, "aryl" refers to aromatic carbocycles and aromatic heterocycles. The term "aryl" refers to an aromatic radical, and includes monoradicals, diradicals (arylene), and higher-radicals. It is preferred that aryl moieties are aromatic carbocycles. "Substituted aryl" refers to any aryl moiety having one or more of its hydrogens replaced with one or more substituents chosen from halogen, C₁₋₆-alkyl, halo C₁₋₆-alkyl, C₁₋₆-alkoxy, halo C₁₋₆-alkoxy, phenyl, and phenoxy, preferably from halogen, C₁₋₆-alkyl, halo C₁₋₄-alkyl, C₁₋₆-alkoxy, halo C₁₋₄-alkoxy, and phenyl, and more preferably from halogen, C₁₋₆-alkyl, C₁₋₆-alkoxy, phenyl, and phenoxy. Preferably, a substituted aryl has from 1 to 3 substituents, and more preferably 1 or 2 substituents. As used herein, the term "polymer" includes oligomers. "Polymer" and "resin" are used interchangeably. The term "oligomer" refers to dimers, trimers, tetramers and other polymeric materials that are capable of further curing. By the term "curing" is meant any process, such as polymerization or condensation, that increases the overall molecular weight of the present oligomers. "Curable" refers to any material capable of being cured under certain conditions.

As used herein, the term "polyarylenes", "polyarylene polymers", and "polyarylene resins" are used interchangeably and refer to polymers having di- or higher-valent, preferably divalent, aryl moieties in the polymer backbone, and may optionally contain one or more divalent linking groups in the polymer backbone. Preferably, the aryl moieties in the polyarylene backbone are divalent. Suitable divalent linking groups include O, S, S(=O), S(=O)₂, C(=O), C(R^{a})₂, Si(R^{b})₂, C₅₋₃₀-aryl, and combinations thereof, wherein each R^{a} is independently chosen from H, C₁₋₂₀-alkyl, and C₅₋₃₀-aryl, and each R^{b} is independently chosen from H, C₁₋₂₀-alkyl, and C₅₋₃₀-aryl. "Polyarylenes" and "polyphenylenes" are used interchangeably herein.

Compositions of the present invention comprise: one or more polyarylene polymers having a backbone comprising as repeating units one or more aryl moieties having one or more epoxy-reactive moieties, and comprising as polymerized units one or more polyalkynyl-substituted aryl first monomers and one or more biscyclopentadienone second monomers; and one or more epoxide-containing crosslinkers. The polyarylene polymers of the invention are prepared using a Diels-Alder reaction of the first and second monomers. The one or more epoxy-reactive moieties may be present on an aryl moiety of the polyalkynyl-substituted aryl first monomer, or on the biscyclopentadienone second monomer, or on both the first monomer and the second monomer. Preferably, the epoxy-reactive moiety is attached (that is, covalently bound) to an aryl moiety of the polyalkynyl-substituted aryl first monomer. As used herein, the term "epoxy-reactive moiety" refers to any moiety having one or more epoxy-reactive substituents.

"Epoxy-reactive substituent" refers to any substituent or functional group that reacts with an epoxy moiety of the epoxy-containing crosslinker under the curing conditions used to form a crosslinked polyarylene film. Preferred epoxy-reactive substituents are carboxyl (-CO₂H), hydroxyl (-OH), thiol (-SH), amino, and combinations thereof. Suitable amino moieties useful as epoxy-reactive substituents of the invention have the formula - NR²⁰R²¹, wherein R²⁰ and R²¹ are independently chosen from H, C₁₋₁₀-alkyl, C₆₋₁₀-aryl, and C₇₋₂₀-aralkyl. Preferably, at least one of R²⁰ and R²¹ is H or C₁₋₄ alkyl, more preferably H, and even more preferably each of R²⁰ and R²¹ is H. The one or more epoxy-reactive substituents may be directly bound to an aryl moiety of the polyarylene oligomer or may be connected to the aryl moiety through a polyvalent, preferably divalent, trivalent, or tetravalent, linking group. Any suitable linking group may be used, such as a C₁₋₄₀-organic residue which may optionally contain one or more heteroatoms chosen from oxygen, nitrogen, sulfur, and combinations thereof. It will be appreciated that one or more epoxy-reactive substituents may be connected to the aryl moiety through a single polyvalent linking group. It will also be appreciated that one or more epoxy-reactive substituents may be bound directly to the aryl moiety and one or more epoxy-reactive substituents may be connected to the aryl moiety through a linking group. It will be further appreciated that when no linking group is present the epoxy-reactive substituent is the epoxy-reactive moiety.

Preferred epoxy-reactive moieties are those of the formula (2) 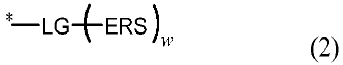 where LG is a linking group or a single chemical bond; each ERS is an epoxy-reactive substituent; *w* is an integer from 1 to 6; and * is the point of attachment to an aryl moiety. Each ERS is preferably chosen from -CO₂H, -OH, -SH, and -NR²⁰R²¹, wherein R²⁰ and R²¹ are as defined above. It is more preferred that each ERS is chosen from -CO₂H, -OH, and - NR²⁰R²¹, and yet more preferably from -CO₂H and -OH. It is preferred that *w* = 1 to 5, more preferably 1 to 3, and still more preferably 1 or 2. When LG is a linking group, it is preferably a divalent linking group. It is preferred that LG is a chemical bond or a divalent organic radical having 1 to 40 carbon atoms, and more preferably a chemical bond or an organic radical having 1 to 30 carbon atoms. The organic radical of LG may optionally contain one or more heteroatoms chosen from O, S, N, and combinations thereof, and preferably contains from 0 to 10 heteroatoms, more preferably from 0 to 8 heteroatoms, and still more preferably from 1 to 8 heteroatoms. Exemplary linking groups for LG include, but are not limited to: C₁₋₄₀-alkyl; optionally substituted C₅₋₄₀-aryl; optionally substituted C₅₋₄₀-aryloxy; optionally substituted C₅₋₄₀-arylamino and combinations thereof. It is preferred that LG is chosen from C₁₋₃₀-alkyl; optionally substituted C₅₋₃₀-aryl; optionally substituted C₅₋₃₀-aryloxy; optionally substituted C₅₋₃₀-arylamino; and combinations thereof, and more preferably C₁₋₃₀-alkyl; optionally substituted C₆₋₂₀-aryl; optionally substituted C₆₋₂₀-aryloxy; optionally substituted C₆₋₂₀-arylamino; and combinations thereof. As used herein, "optionally substituted" aryl, aryloxy or arylamino refers to both unsubstituted aryl, aryloxy or arylamino, and aryl, aryloxy or arylamino having one or more of its aromatic hydrogens replaced with one or more substituents chosen from halogen, C₁₋₆-alkyl, C₁₋₆-alkoxy, and C₅₋₁₀-aryl.

Exemplary epoxy-reactive moieties of formula (2) include, without limitation: - CO₂H; -OH; -NR²⁰R²¹ wherein R²⁰ and R²¹ are as described above; mono-, di-, tri- and tetra-hydroxy-C₁₋₃₀-alkyl; mono-, di-, tri-, and tetra-hydroxy-C₅₋₃₀-aryl; mono-, di-, tri-, and tetra-hydroxy-C₅₋₃₀-aryloxy; mono-, di-, tri-, and tetra-hydroxy-C₅₋₃₀-arylamino; mono-, di-, tri-, and tetra-amino-C₁₋₃₀-alkyl; mono-, di-, tri-, and tetra-amino-C₅₋₃₀-aryl; mono-, di-, tri-, and tetra-amino-C₅₋₃₀-arylamino; mono-, di-, tri-, and tetra-carboxy-C₅₋₃₀-aryl; mono-, di-, tri-, and tetra-carboxy-C₁₋₃₀-alkyl; mono-, di-, tri-, and tetra-hydroxy-C₁₋₁₀-alkyl esters of carboxy-C₁₋₃₀-alkyl such as HO-C₁₋₁₀-alkyl-O-C(=O)-C₁₋₃₀-alkyl; mono-, di-, tri-, and tetra-amino-C₁₋₁₀-alkyl amides of carboxy-C₁₋₃₀-alkyl such as H₂N-C₁₋₁₀-alkyl-NH-C(=O)-C₁₋₃₀-alkyl; carboxylic acid-containing esters of alkyl carboxylates, such as those of the formula HO-C(=O)-C₁₋₃₀-alkyl-O-C(=O)-C₁₋₃₀-alkyl; and the like. Suitable mono-, di-, tri- and tetra-hydroxy-C₁₋₃₀-alkyl moieties include, but are not limited to: mono-, di-, tri-, and tetra-hydroxy-alkyleneoxy and mono-, di-, tri-, and tetra-hydroxy-C₁₋₁₀-alkylamino-C₁₋₁₀-alkyl. Suitable mono-, di-, tri-, and tetra-carboxy-C₁₋₃₀-alkyl moieties, include, without limitation, mono-, di-, tri-, and tetra-carboxy-poly(C₂₋₄-alkyleneoxy), and mono-, di-, tri-, and tetra-carboxy-C₁₋₃₀-alkylaminoalkyl.

Suitable epoxy-reactive moieties include, without limitation: -OH; -C(=O)-OH; - NR²⁰R²¹; -C₁₋₃₀-alkyl-(C(=O)-OH)₁₋₃ such as carboxymethyl (-CH₂C(=O)-OH), carboxyethyl (-C₂H₄C(=O)-OH), carboxypropyl (-(CH₂)₃C(=O)-OH), -(OC₂H₂)₁₋₁₀-C(=O)-OH, -(OC₃H₆)₁₋₁₀-C(=O)-OH, and -(O(C₄H₈)₁₋₁₀-C(=O)-OH; -C₁₋₃₀-alkyl-(OH)₁₋₃ such as hydroxymethyl, hydroxyethyl, hydroxypropyl, dihydroxypropyl, hydroxycyclohexyl, hydroxycyclopentyl, - (OC₂H₂)₁₋₁₀-OH, -(OC₃H₆)₁₋₁₀-OH, and -(O(C₄H₈)₁₋₁₀-OH; -C₅₋₃₀-aryl-(OH)₁₋₄ such as hydroxyphenyl, dihydroxyphenyl, trihydroxyphenyl, hydroxyquinolyl hydroxynaphthyl, dihydroxynaphthyl, hydroxybiphenyl, dihydroxybiphenyl, and hydroxypyridyl; -O-C₅₋₃₀₋aryl-(OH)₁₋₄ such as hydroxyphenoxy, dihydroxyphenoxy, trihydroxyphenoxy, hydroxynaphthyloxy and dihydroxynaphthyloxy; -C₅₋₃₀-aryl-(C(=O)OH)₁₋₄ such as pyridine carboxylic acid, benzene carboxylic acid, benzene dicarboxylic acid, naphthalene carboxylic acid, and naphthalene dicarboxylic acid; (HO-C₁₋₂₀-alkyl)₁₋₂-NR²²₀₋₁- such as hydroxyethylamino, hydroxypropylamino, di(hydroxyethyl)-amino, and di(hydroxypropyl)amino; (HO-C₁₋₂₀-alkyl)₁₋₂-NR²²₀₋₁ -C₁₋₂₀-alkyl such as hydroxyethylaminomethyl (HO-C₂H₄-NH-CH₂-), hydroxyethylaminoethyl (HO-C₂H₄-NH-C₂H₄-), hydroxyethylyaminopropyl (HO-C₂H₄-NH-(CH₂)₃-), hydroxypropylaminopropyl, di(hydroxyethyl)aminoethyl, di(hydroxyethyl)aminopropyl, hydroxyethylaminoethoxy, di(hydroxyethyl)aminoethoxy, hydroxypropylaminopropoxy, and di(hydroxypropyl)aminopropoxy; (R²⁰R²¹N)₁₋₃-C₁₋₃₀-alkyl such as aminomethyl, aminoethyl, aminopropyl, methylaminoethyl, dimethylaminopropyl, ethylmethylaminopropyl, aminoethoxy, dimethylaminoethoxy, aminopropoxy, and ethylmethylaminoethoxy; (R²⁰R²¹N-C₁₋₂₀-alkyl)₁₋₂-NR²²₀₋₁- such as aminoethylamino, (N-methylaminoethyl)amino, aminopropylamino, di(aminoethyl)amino, and di(aminopropyl)amino; R²⁰R²¹N-C₁₋₂₀alkyl-O- such as aminomethoxy (H₂N-CH₂-O-), aminoethoxy aminopropoxy, and N-methylaminopropoxy; (R²⁰R²¹N)₁₋₃-C₅₋₃₀-aryl such as aminophenyl, aminopyridyl, aminonaphthyl, diaminophenyl, methylaminophenyl, diaminonaphthyl, and aminobiphenyl; (HO-C(=O)-C₁₋₃₀-alkyl)₁₋₂-NH₀₋₁- such as carboxymethylamino, carboxyethylamino; carboxyethyl(N-methyl)amino, carboxypropylamino bis-(carboxymethyl)amino, and bis(carboxyethyl)amino; (HO-C(=O)-C₁₋₃₀-alkyl)₁₋₂-NH₀₋₁-alkyl such as carboxymethylaminomethyl, carboxyethylaminoethyl, carboxypropyl-aminoethyl, carboxyethylaminoethylamino, bis(carboxymethyl)aminoethyl, bis(carboxymethyl)aminoethylamino, carboxypropylaminoethylamino, bis(carboxypropyl)-aminopropylamino, and bis(carboxyethyl)aminoethylamino; HO-C₁₋₃₀-alkyl-O-C(=O)-C₁₋₃₀-alkyl such as hydroxyethyl carboxyethyl, -(OC₂H₂)₁₋₁₀-C(=O)-C₁₋₁₀-alkyl-OH, -(OC₃H₆)₁₋₁₀-C(=O)-C₁₋₁₀-alkyl-OH, and -(O(C₄H₈)₁₋₁₀-C(=O)-C₁₋₁₀-alkyl-OH; HO-C(=O)-C₁₋₃₀-alkyl-O-C(=O)-C₁₋₃₀-alkyl; and the like; wherein R²⁰ and R²¹ are as described above; and R²² = H, C₁₋₁₀-alkyl, or C₆₋₁₀-aryl.

Suitable first monomers useful in the preparation of the present polyarylenes may be any polyalkynyl-substituted aryl, that is, any aryl moiety having two or more alkynyl substituents bound directly to the aryl moiety. When a biscyclopentadienone second monomer containing an aryl moiety having one or more epoxy-reactive moieties is used, the polyalkynyl-substituted aryl first monomer does not need to contain an epoxy-reactive moiety. When a biscyclopentadienone second monomer containing an aryl moiety that does not have one or more epoxy-reactive moieties is used, the polyalkynyl-substituted aryl first monomer must contain one or more epoxy-reactive moieties. It is preferred that the polyalkynyl-substituted aryl first monomer comprises an aryl moiety having one or more epoxy-reactive moieties. Suitable polyalkynyl-substituted aryl first monomers have the general formula (1) 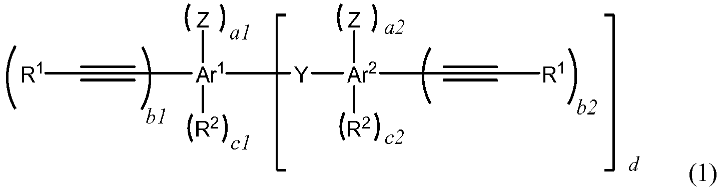 wherein Ar¹ and each Ar² are independently a C₅₋₃₀-aryl moiety; each R¹ is independently chosen from H, C₅₋₃₀-aryl, and substituted C₅₋₃₀-aryl; each R² is independently chosen from C₁₋₁₀-alkyl, C₁₋₁₀-haloalkyl, C₁₋₁₀-alkoxy, CN, and halo; each Z is an epoxy-reactive moiety; Y is a single chemical bond or a divalent linking group chosen from -O-, -S-, -S(=O)-, - S(=O)₂-, -C(=O)-, -(C(R⁵)₂)*_{z}*-, C₅₋₃₀ aryl, and -(C(R⁵)₂)*_{z1}*-(C₅₋₃₀ aryl)-(C(R⁵)₂)_{z*2*}-; each R⁵ is independently chosen from H, hydroxy, halo, C₁₋₁₀-alkyl, C₁₋₁₀-haloalkyl, and C₅₋₃₀-aryl; *a1* = 0 to 3; each *a2* = 0 to 3; *b1 =* 1 to 4; each *b2 =* 0 to 2; *c1* = 0 to 2; each *c2* = 0 to 2; *a1* + each *a2* = 1 to 6; *b1* + each *b2* = 2 to 6; *c1* + each *c2* = 0 to 6; *d* = 0 to 2; *z* = 1 to 10; *z1* = 0 to 10; *z2* = 0 to 10; and *z1* + *z2* = 1 to 10. Each R¹ is preferably independently chosen from H and C₆₋₂₀-aryl, more preferably from H and C₆₋₁₀-aryl, and yet more preferably from H and phenyl. It is preferred that each R² is independently chosen from C₁₋₁₀-alkyl, C₁₋₁₀-haloalkyl, C₁₋₁₀-alkoxy, and halo, and more preferably from C₁₋₁₀-alkyl, C₁₋₁₀-haloalkyl, and halo. Preferably, Y is a single chemical bond or a divalent linking group chosen from -O-, -S-, - S(=O)-, -S(=O)₂-, -C(=O)-, -(C(R⁵)₂)*_{z}*-, and C₅₋₃₀-aryl, and more preferably a chemical bond, -O-, -S-, -S(=O)₂-, -C(=O)-, and -(C(R⁵)₂)*_{z}*-. R⁵ is preferably H, halo, C₁₋₁₀-alkyl, halo C₁₋₁₀-alkyl, and C₅₋₃₀-aryl, and more preferably fluoro, C₁₋₆-alkyl, fluoro C₁₋₆-alkyl, and C₆₋₂₀-aryl. Preferably, *a1* = 1 to 3, more preferably 1 to 2, and most preferably *a1* = 1. It is preferred that each *a2* = 0 to 2. Preferably, *a1* + each *a2 =* 1 to 4, more preferably 1 to 3, and yet more preferably 1 to 2. It is preferred that *b1 =* 1 to 2, and more preferably 2. It is preferred that each *b2 =* 0 or 1. Preferably, *b1* + each *b2* = 2 to 4, and more preferably 2 or 3, and even more preferably 2. Preferably, *c1* = 0 or 1, and more preferably 0. It is preferred that each *c2* is 0 or 1, and more preferably 0. Preferably, *c1* + each *c2* is 0 to 3, more preferably 0 to 2, and even more preferably 0. It is preferred that *d* = 0 or 1, and more preferably 0. Preferably, *z* = 1 to 6, more preferably 1 to 3, and even more preferably *z* = 1. Preferably, *z1* and *z2* are each 0 to 5. It is preferred that *z1* + *z2* = 1 to 6, and more preferably 2 to 6. It is preferred that Z comprises one or more epoxy-reactive substituents chosen from -CO₂H, - OH, -SH, -NR²⁰R²¹, and combinations thereof, wherein R²⁰ and R²¹ are independently chosen from H, C₁₋₁₀-alkyl, C₆₋₁₀-aryl, and C₇₋₂₀-aralkyl. Optionally, Z may further comprise a linking group disposed between the one or more epoxy-reactive substituents and the aryl moiety. Such linking group is preferably a C₁₋₄₀-organic residue which may optionally contain one or more heteroatoms chosen from oxygen, nitrogen, sulfur, and combinations thereof. More preferably, each Z has the formula (2) 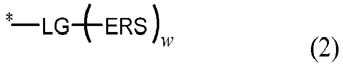 wherein LG, ERS, *w* and * are as defined above.

Suitable aryl moieties for Ar¹ and Ar² include, but are not limited to, pyridyl, phenyl, naphthyl, anthracenyl, phenanthryl, tetracenyl, pyrenyl, perylenyl, coronenyl, pentacenyl, triphenylenyl, tetraphenyl, benzotetracenyl, biphenyl, binaphthyl, diphenyl ether, and dinaphthyl ether. It is preferred that Ar¹ and each Ar² in formula (1) are independently a C₆₋₃₀-aryl moiety. Preferred aryl moieties for Ar¹ and Ar² are phenyl, naphthyl, anthracenyl, phenanthryl, pyrenyl, tetracenyl, pentacenyl, tetraphenyl, triphenylenyl, and perylenyl.

Preferred first monomers of formula (1) are those of formulas (3) and (4): 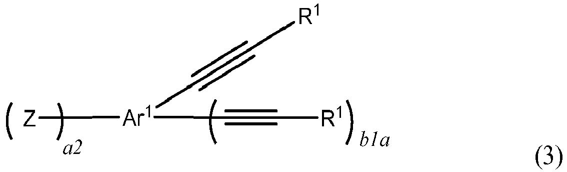 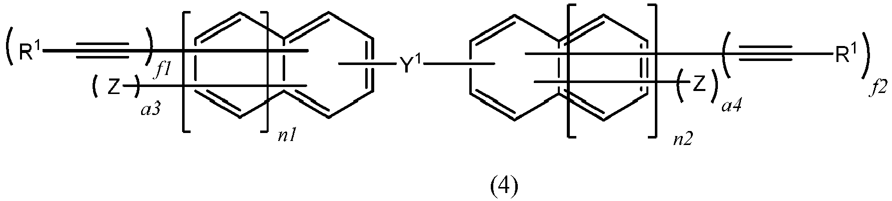 wherein Ar¹, R¹, and Z are as defined above for formula (1); *a2* is 1 to 4; *a3* is 1 or 2; *a4* is 0 to 2; *b1a* is 1 to 4; *f1* is 1 to 4; *f2* is 0 to 4; *f1* + *f2* = 2 to 6; each of *n1* and *n2* is independently 0 to 4; and Y¹ is a single chemical bond, O, S, S(=O)₂, C(=O), C(CH₃)₂, CF₂, and C(CF₃)₂. It will be appreciated by those skilled in the art that the brackets ("[ ]") in formula (4) refer to the number of aromatic rings fused to the phenyl ring. Accordingly, when *n1* (or *n2*) = 0, the aromatic moiety is phenyl; when *n1* (or *n2*) = 1, the aromatic moiety is naphthyl; when *n1* (or *n2*) = 2, the aromatic moiety may be anthracenyl or phenanthryl; when *n1* (or *n2*) = 3, the aromatic moiety may be tretacenyl, tetraphenyl, triphenylenyl, or pyrenyl; and when *n1* (or *n2*) = 4, the aromatic moiety may be perylenyl or benzotetracenyl. In formula (3), *a2* is preferably 1 to 2, and more preferably *a2* = 1. It is preferred that *b1a* in formula (3) is 1 or 2, and more preferably 1. R¹ is preferably H or phenyl. Ar¹ in formula (3) is preferably phenyl, naphthyl, anthracenyl, phenanthryl, pyrenyl, tetracenyl, pentacenyl, tetraphenyl, triphenylenyl, and perylenyl. In formula (4), it is preferred that *n1* and *n2* are independently chosen from 0, 1,3, and 4, more preferably from 0, 1 and 3, and even more preferably from 1 and 3. It is further preferred that *n1* = *n2*. In formula (4), Y¹ is preferably a chemical bond, O, S(=O)₂, C(=O), C(CH₃)₂, CF₂, and C(CF₃)₂, and more preferably a chemical bond.

Particularly preferred monomers of formula (3) are monomers of formulas (5) to (9): 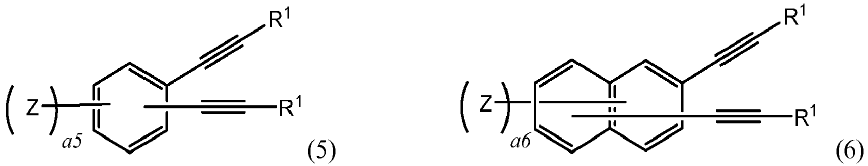 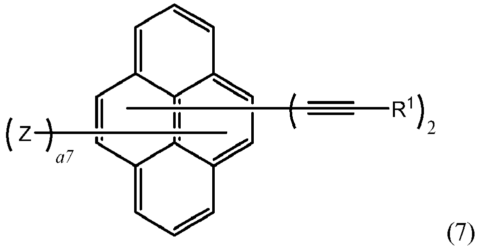 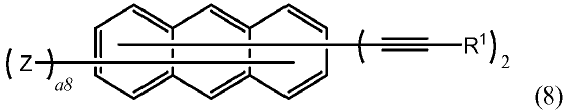 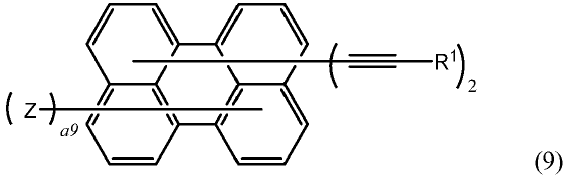 wherein R¹ and Z are as described above for formula (1); and each of *a5*, *a6*, *a7*, *a8* and *a9* is independently 1 to 4. Preferably, *a5* = 1 to 3, more preferably 1 or 2, and yet more preferably 1. It is preferred that *a6* is 1 to 3, more preferably 1 or 2, and even more preferably 1. Preferably, each of *a7* to *a9* is independently 1 to 3, and more preferably 1 to 2.

In the monomers of any of the above formulas (1), and (3) to (9), any two alkynyl moieties on the same aromatic ring may have any relationship to each other, such as an ortho, meta or para relationship. When two alkynyl moieties are on the same aromatic ring, it is preferred that they have a meta or para relationship to each other. Preferably, the alkynyl moieties in the monomers of formulas (1) and (3) to (9) do not have an ortho relationship to each other. Suitable polyalkynyl-substituted aryl first monomers are generally commercially available or may be readily prepared by methods known in the art. Particularly preferred first monomers are: 1,3-diethynylbenzene carboxylic acid; 1,4-diethynylbenzene carboxylic acid; 1,3-bis(phenylethynyl)benzene carboxylic acid; 1,4-bis(phenylethynyl)benzene carboxylic acid; 1,3-diethynylphenol; 1,4-diethynylphenol; 1,3-bis(phenylethynyl)phenol; 1,4-bis(phenylethynyl)phenol; hydroxyethyl 1,3-diethynylbenzenecarboxylate; hydroxyethyl 1,4-diethynylbenzenecarboxylate; hydroxyethyl 1,3-bis(phenylethynyl)benzenecarboxylate; hydroxyethyl 1,4-bis(phenylethynyl)benzenecarboxylate; polyethyleneoxy esters of 1,3-diethynylbenzene carboxylic acid; polyethyleneoxy esters of 1,4-diethynylbenzene carboxylic acid; polyethyleneoxy esters of 1,3-bis(phenylethynyl)benzene carboxylic acid; and polyethyleneoxy esters of 1,4-bis(phenylethynyl)benzene carboxylic acid.

The present polyarylene polymers may be comprised of one polyalkynyl-substituted aryl first monomer, or a mixture of two or more such monomers. Monomers of formula (3) are preferred first monomers. It is preferred that the present polyarylene polymers are comprised of polymerized units of one or more polyalkynyl-substituted aryl first monomers of formula (3). In an alternate preferred embodiment, the present polymers are comprised of polymerized units of one or more monomers of formula (4), or in yet another alternate embodiment of one or more monomers of formula (3) and one or more monomers of formula (4). Mixtures of polymers comprising as polymerized units one or more monomers of formula (1) may suitably be used in the present compositions. It will be appreciated that a combination of one or more polyalkynyl-substituted aryl first monomers having one or more epoxy-reactive moieties and one or more polyalkynyl-substituted aryl first monomers that are free of epoxy-reactive moieties may suitably be used to prepare the present polyarylene polymers. Preferably, a combination of one or more polyalkynyl-substituted aryl first monomers having one or more epoxy-reactive moieties and one or more polyalkynyl-substituted aryl first monomers that are free of epoxy-reactive moieties may suitably be used to prepare the present polyarylene polymers.

Suitable polyalkynyl-substituted aryl first monomers that are free of epoxy-reactive moieties are those described in formulas (1) and (3) to (9) above wherein each of *a1* to *a9* is 0. Preferred polyalkynyl-substituted aryl first monomers that are free of epoxy-reactive moieties are those of formula (10) 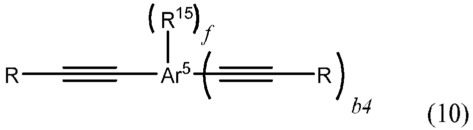 wherein each R is as defined above for the monomers of formula (1); Ar⁵ is a C₅₋₃₀ aromatic moiety; each R¹⁵ is independently chosen from C₁₋₄ alkyl, C₁₋₄ haloalkyl, C₁₋₄ alkoxy, optionally substituted C₇₋₁₄ aralkyl, and optionally substituted C₆₋₁₀ aryl; *b4* = 1 or 2; and *f* = 0 to 4. "Substituted aralkyl" refers to an aralkyl moiety, such as benzyl or phenethyl, having one or more of its hydrogens replaced with one or more substituents chosen from halogen, C₁₋₆-alkyl, C₁₋₆-haloalkyl, C₁₋₆-alkoxy, C₁₋₆-haloalkoxy, phenyl, and phenoxy, preferably from halogen, C₁₋₆-alkyl, C₁₋₄-haloaₗkyl, C₁₋₆-alkoxy, C₁₋₄-haloalkoxy, and phenyl, and more preferably from halogen, C₁₋₆-alkyl, C₁₋₆-alkoxy, phenyl, and phenoxy. Fluorine is the preferred halogen. In formula (10), it is preferred that each R is independently H or C₆₋₁₀-aryl, and more preferably H or phenyl. It is preferred that each R¹⁵ is independently chosen from C₁₋₄-alkyl, C₁₋₄-fluoroaₗkyl, C₁₋₄-alkoxy, benzyl, phenethyl, phenyl, naphthyl, substituted phenyl and substituted naphthyl, more preferably C₁₋₂-alkyl, C₁₋₄-fluoroalkyl, C₁₋₂-alkoxy, phenyl, and substituted phenyl, and yet more preferably from C₁₋₂-alkyl, C₁₋₄-fluoroalkyl, C₁₋₂-alkoxy, and phenyl. Preferably, *b4* = 2. Preferably, *f* = 0 to 3, more preferably 0 to 2, and yet more preferably *f* = 0. Ar⁵ may be any suitable C₅₋₃₀-aromatic moiety, such as, without limitation, phenyl, naphthyl, anthracenyl, phenanthryl, tetracenyl, pyrenyl, perylenyl, coronenyl, pentacenyl, triphenylenyl, tetraphenyl, benzotetracenyl, biphenyl, binaphthyl, diphenyl ether, and dinaphthyl ether. Preferably, monomers of formula (10) comprise 2 or 3 alkynyl moieties having a terminal hydrogen or terminal phenyl moiety. Any 2 alkynyl moieties in the monomers of formula (10) may have an ortho, meta or para relationship to each other, and preferably a meta or para relationship to each other. Preferably, the alkynyl moieties do not have an ortho relationship to each other. A single monomer of formula (10) may be used to prepare the present polymers, or two or more monomers of formula (10) but different from each other may be used. When a single monomer of formula (10) is used, it is preferred that *b4* = 2. In one preferred embodiment, the present polymers further comprise as polymerized units a monomer of formula (10), and more preferably a monomer of formula (10) wherein *b4* = 2. In an alternate preferred embodiment, the present polymers further comprise as polymerized units one monomer of formula (10) wherein *b4* = 1, and another monomer of formula (10) wherein *b4* = 2.

Compounds useful as the polyalkynyl-substituted aryl first monomers that are free of epoxy-reactive moieties of formula (10) are generally commercially available, or may be prepared by methods known in the art. Preferred monomers of formula (10) are: 1,3-diethynylbenzene; 1,4-diethynylbenzene; 4,4'-diethynyl-1,1'-biphenyl; 3,5-diethynyl-1,1'-biphenyl; 1,3,5-triethynylbenzene; 1,3-diethynyl-5-(phenylethynyl)benzene; 1,3-bis(phenylethynyl)benzene; 1,4-bis(phenylethynyl)benzene; 1,3,5-tris(phenylethynyl)-benzene; 4,4'-bis(phenylethynyl)-1,1'-biphenyl; 4,4'-diethynyl-diphenylether; and mixtures thereof. More preferably, the monomers of formula (10) are chosen from: 1,3-diethynylbenzene; 1,4-diethynylbenzene; 1,3,5-triethynylbenzene; 4,4'-diethynyl-1,1'-biphenyl; 1,3-bis(phenylethynyl)-benzene; 1,4-bis(phenylethynyl)benzene; 4,4'-bis(phenylethynyl)-1,1'-biphenyl; and mixtures thereof. Even more preferably, the monomers of formula (10) are chosen from: 1,3-diethynylbenzene; 1,4-diethynylbenzene; 4,4'-diethynyl-1,1'-biphenyl; 1,3,5-triethynylbenzene; and mixtures thereof.

Any monomer containing two cyclopentadienone moieties may suitably be used as the second monomer to prepare the polyarylene polymers of the invention. A mixture of 2 or more different monomers, each having two cyclopentadienone moieties, may be used as the second monomer. Such monomers containing two cyclopentadienone moieties are well-known in the art, such as those described in U.S. Pat. Nos. 5,965,679; 6,288,188; and 6,646,081; and in Int. Pat. Pubs. WO 97/10193 and WO 2004/073824. Suitable biscyclopentadienone second monomers may have one or more epoxy-reactive moieties attached to an aryl moiety. Any of the epoxy-reactive moieties described above for the polyalkynyl-substituted aryl first monomers may be used in the biscyclopentadienone second monomers. It is preferred that the biscyclopentadienone second monomers do not have epoxy-reactive moieties. It will be appreciated that a combination of one or more biscyclopentadienone second monomers having one or more epoxy-reactive moieties and one or more biscyclopentadienone second monomers that are free of epoxy-reactive moieties may suitably be used to prepare the present polyarylene polymers.

Preferred biscyclopentadienone second monomers have the structure shown in formula 11 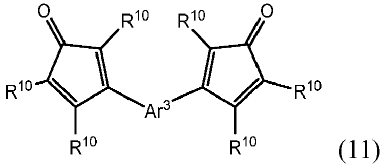 wherein each R¹⁰ is independently chosen from H, C₁₋₆ alkyl, or optionally substituted C₅₋₃₀ aryl; and Ar³ is an aromatic moiety. When such biscyclopentadienone second monomers contain one or more epoxy-reactive moieties, such moieties are typically present on Ar³ or on the aryl moiety of R¹⁰ or both on Ar³ and the aryl moiety of R¹⁰. That is, when R¹⁰ is a substituted aryl, such substituted aryl also includes an aryl having one or more epoxy-reactive moieties described above for formula (1). Preferably, each R¹⁰ is independently chosen from C₃₋₆-alkyl, C₆₋₁₀-aryl and substituted C₆₋₁₀-aryl, more preferably each R¹⁰ is phenyl or substituted phenyl, and even more preferably phenyl. A wide variety of aromatic moieties are suitable for use as Ar³, such as those disclosed in U.S. Pat. No. 5,965,679. Exemplary aromatic moieties useful for Ar³ include those having the structure shown in formula (12) 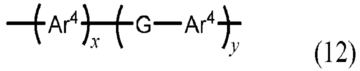 wherein *x* is an integer chosen from 1, 2 or 3; *y* is an integer chosen from 0, 1, or 2; each Ar⁴ is independently chosen from 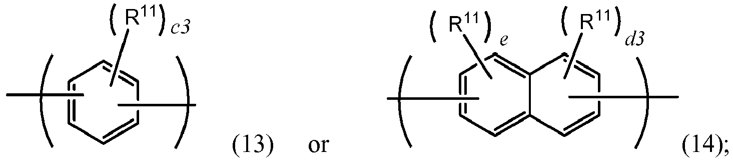 each R¹¹ is independently chosen from halogen, C₁₋₆-alkyl, halo C₁₋₆-alkyl, C₁₋₆-alkoxy, halo C₁₋₆-alkoxy, phenyl, and phenoxy; *c3* is an integer from 0 to 4; each of *d3* and *e* is independently an integer from 0 to 3; each G is independently chosen from O, S, NR¹², PR¹², P(=O)R¹², C(=O), CR¹³R¹⁴, and SiR¹³R¹⁴; R¹², R¹³, and R¹⁴ are independently chosen from H, C₁₋₄-alkyl, halo C₁₋₄-alkyl, and phenyl. It is preferred that *x* is 1 or 2, and more preferably 1. It is preferred that *y* is 0 or 1, and more preferably 1. Preferably, each R¹¹ is independently chosen from halogen, C₁₋₄-alkyl, halo C₁₋₄-alkyl, C₁₋₄-alkoxy, halo C₁₋₄-alkoxy, and phenyl, and more preferably from fluoro, C₁₋₄-alkyl, fluoro C₁₋₄-alkyl, C₁₋₄-alkoxy, fluoro C₁₋₄-alkoxy, and phenyl. It is preferred that *c3* is from 0 to 3, more preferably from 0 to 2, and yet more preferably 0 or 1. It is preferred that each of *d3* and *e* is independently 0 to 2, and more preferably 0 or 1. In formula (14), it is preferred that *d3* + *e* = 0 to 4, and more preferably 0 to 2. Each G is preferably independently chosen from O, S, NR¹², C(=O), CR¹³R¹⁴, and SiR¹³R¹⁴, more preferably from O, S, C(=O), and CR¹³R¹⁴, and yet more preferably from O, C(=O), and CR¹³R¹⁴. It is preferred that each R¹², R¹³, and R¹⁴ are independently chosen from H, C₁₋₄-alkyl, fluoro C₁₋₄-alkyl, and phenyl; and more preferably from H, C₁₋₄-alkyl, fluoro C₁₋₂-alkyl, and phenyl. Preferably, each Ar⁴ has the formula (13).

Optionally, one or more end capping monomers may be used to prepare the present polyarylene polymers. Such end capping monomers have a single alkyne moiety and a solubility improving polar group and which function to cap one end, preferably two ends, and more preferably all ends, of the present polymers. Suitable end capping monomers are those disclosed in U.S. Pat. App. Publication No. 2016/0060393 (Gilmore et al.). It will be appreciated by those skilled in the art that reaction conditions can be selected such that these optional end capping monomers preferentially react with alkynyl moieties having terminal hydrogens (R = H) in the polymer over alkynyl moieties having terminal aryl moieties (R = C₆₋₂₀-aryl). Preferably, the polar moieties present in these optional end capping monomers are cleavable under conditions used to cure the present polyarylene polymers. Suitable optional end capping monomers are those of formula (15): 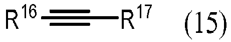 wherein R¹⁶ is H, optionally substituted C₁₋₁₀-alkyl, optionally substituted C₇₋₁₂-aralkyl, optionally substituted C₆₋₁₀-aryl, or R¹⁷; and R¹⁷ is a polar moiety. Suitable polar moieties are any hydrocarbyl moiety having from 1 to 20 carbon atoms and one or more functional groups chosen from -C(=O)-R¹⁸, -C(=O)OR¹⁸, -OH, -NO₂, and -NR¹⁸R¹⁹, where R¹⁸ and R¹⁹ are independently chosen from H, C₁₋₁₀-alkyl, C₇₋₁₆-aralkyl, and C₆₋₁₀-aryl. Preferably, the polar moiety is chosen from -C(=O)-R¹⁸, -C(=O)OR¹⁸, -OH, and -NR¹⁸R¹⁹, and more preferably from -C(=O)-R¹⁸, -C(=O)OR¹⁸, and -OH. Such -C(=O)-, -OH, and -NR¹⁸R¹⁹ functional groups may be part of another functional group, as in carboxylic acids, anhydrides, amides, ketones, esters, and the like. It is preferred that the polar moiety is chosen from carboxyl, C₂₋₁₂-aliphatic carboxylate, hydroxy C₁₋₁₀-alkyl, hydroxy C₆₋₁₀-aryl, C₇₋₂₀-aryl carboxylic acid, C₈₋₂₀-aryl carboxylic acid anhydride, C₇₋₂₀-aryl carboxylates, C₇₋₂₀-aryl amide, C₈₋₂₀-aryl imide, amino C₁₋₁₀-alkyl, and C₆₋₂₀-aryl amine. More preferably, the polar moiety is chosen from carboxyl, C₂₋₁₂-aliphatic carboxylate, hydroxy C₁₋₁₀-alkyl, hydroxy C₆₋₁₀-aryl, C₇₋₁₆-aryl carboxylic acid, and C₈₋₁₆-aryl carboxylic acid anhydride. It will be appreciated by those skilled in the art that when the end-capping monomer comprises -OH, -C(=O)-OH, or NR¹⁸R¹⁹ as a polar moiety, such polar moiety may also function as an epoxy-reactive moiety. Exemplary end capping monomers are: propiolic acid; acetylene dicarboxylic acid; phenyl propiolic acid; ethynyl benzoic acid; ethynyl phthalic acid; propargyl alcohol; propargylamine; 2-butyn-1,4-diol; 2-methyl-3-butyn-2-ol; 3-butyn-1-ol; 3-butyn-2-ol; 2-butyn-1-ol; 2-butynoic acid; ethynyl phenol; xylityl propiolate; ethynyl phthalic anhydride; ethynyl phthalimide; ethynyl benzamide; 2-butyn-1,4-diol diacetate; 3-butyn-2-one; 1-ethynyl-1-cyclohexanol; 1-ethynylcyclohexylamine; 1-ethynylcyclopentanol; ethynylaniline; N-(ethynylphenyl)acetamide; 2-carbamoyl-5-ethynylbenzoic acid; ethynyl-nitrobenzene; propiolamide; N-hydroxyl-propiolamide; 2-aminobut-3-ynoic acid; and mixtures thereof. Preferred end capping monomers are: propiolic acid; acetylene dicarboxylic acid; phenyl propiolic acid; ethynyl benzoic acid; ethynyl phthalic acid; propargyl alcohol; 2-butyn-1,4-diol; 2-methyl-3-butyn-2-ol; 3-butyn-1-ol; 3-butyn-2-ol; 2-butyn-1-ol; 2-butynoic acid; ethynyl phenol; xylitylpropiolate; ethynyl phthalic anhydride; 2-butyn-1,4-diol diacetate; and mixtures thereof. Such end capping monomers are generally commercially available, or may be prepared by methods known in the art.

The polyarylene polymers of the present invention are prepared by reacting one or more polyalkynyl-substituted aryl first monomers described above, one or more biscyclopentadienone second monomers described above, wherein at least one of the first and second monomers comprises an aryl moiety having one or more epoxy-reactive moieties, and optionally one or more additional monomers, such as the monomers of formulas (10) and/or (15) described above, in a suitable organic solvent. The mole ratio of the total first monomers (that is, polyalkynyl-containing monomers) to the total second monomers (that is, monomers containing two cyclopentadienone moieties) is from 1:1.2 to 1.95:1, preferably from 1:1.15 to 1.75:1, and more preferably from 1:1.1 to 1.2:1. When an end-capping monomer, such as the monomer of formula (15), is used, it is typically used in a total amount of from 0.05 to 0.25 moles, based on 1 mole of the second monomer, preferably from 0.075 to 0.2 moles, and more preferably from 0.09 to 0.125 moles. Suitable organic solvents useful to prepare the present oligomers are benzyl esters of C₂₋₆-alkanecarboxylic acids, dibenzyl esters of C₂₋₆-alkanedicarboxylic acids, tetrahydrofurfuryl esters of C₂₋₆-alkanecarboxylic acids, ditetrahydrofurfuryl esters of C₂₋₆-alkanedicarboxylic acids, phenethyl esters of C₂₋₆-alkanecarboxylic acids, diphenethyl esters of C₂₋₆-alkanedicarboxylic acids, aromatic ethers, aromatic hydrocarbons, cyclic hydrocarbons, carbonates, and lactones. Preferred aromatic ethers are diphenyl ether, dibenzyl ether, C₁₋₆-alkoxy-substituted benzenes and benzyl C₁₋₆-alkyl ethers, and more preferably C₁₋₄-alkoxy-substituted benzenes and benzyl C₁₋₄-alkyl ethers. Preferred organic solvents are benzyl esters of C₂₋₄-alkanecarboxylic acids, dibenzyl esters of C₂₋₄-alkanedicarboxylic acids, tetrahydrofurfuryl esters of C₂₋₄-alkanecarboxylic acids, ditetrahydrofurfuryl esters of C₂₋₄-alkanedicarboxylic acids, phenethyl esters of C₂₋₄-alkanecarboxylic acids, diphenethyl esters of C₂₋₄-alkanedicarboxylic acids, C₁₋₆-alkoxy-substituted benzenes, and benzyl C₁₋₆-alkyl ethers, more preferably benzyl esters of C₂₋₆-alkanecarboxylic acids, tetrahydrofurfuryl esters of C₂₋₆-alkanecarboxylic acids, phenethyl esters of C₂₋₆-alkanecarboxylic acids, C₁₋₄-alkoxy-substituted benzenes, benzyl C₁₋₄-alkyl ethers, dibenzyl ether, carbonates, and lactones, and yet more preferably benzyl esters of C₂₋₆-alkanecarboxylic acids, tetrahydrofurfuryl esters of C₂₋₆-alkanecarboxylic acids, C₁₋₄-alkoxy-substituted benzenes, benzyl C₁₋₄-alkyl ethers, carbonates, and lactones. Exemplary organic solvents include, without limitation, benzyl acetate, benzyl proprionate, tetrahydrofurfuryl acetate, tetrahydrofurfuryl propionate, tetrahydrofurfuryl butyrate, anisole, methylanisole, dimethylanisole, dimethoxybenzene, ethylanisole, ethoxybenzene, xylene, mesitylene, cumene, limonene, benzyl methyl ether, benzyl ethyl ether, and propylene carbonate, and preferably benzyl acetate, benzyl proprionate, tetrahydrofurfuryl acetate, tetrahydrofurfuryl propionate, tetrahydrofurfuryl butyrate, anisole, methylanisole, dimethylanisole, dimethoxybenzene, ethylanisole, ethoxybenzene, xylene, mesitylene, cumene, limonene, propylene carbonate, and gamma-butyrolactone.

The polymers of the present invention may be prepared by combining one or more polyalkynyl-substituted aryl first monomers, one or more biscyclopentadienone second monomers, optionally one or more end capping monomers, optionally one or more polyalkynyl-substituted aryl monomers free of an epoxy-reactive moiety, and organic solvent, each as described above, in any order in a vessel, and heating the mixture. The one or more second monomers may be combined with the organic solvent in a vessel, and then the one or more first monomers and any optional additional monomers are added to the mixture. In one embodiment, the one or more second monomers and organic solvent mixture is heated to the desired reaction temperature before the one or more first monomers are added. The polyalkynyl-substituted aryl first monomer may be added over a period of time, such as from 0.25 to 48 hours, and preferably from 1 to 6 hours, to reduce exotherm formation, but is preferably added at one time. The biscyclopentadienone second monomer and organic solvent mixture may be heated to the desired reaction temperature before the first monomer and any optional monomers are added. Alternatively, the biscyclopentadienone second monomer, first monomer, optional polyalkynyl-substituted aryl monomer free of an epoxy-reactive moiety, optional end capping monomer and solvent are added to a vessel, and then heated to the desired reaction temperature and held at this temperature for a period of time to provide the desired oligomer. The reaction mixture is heated at a suitable temperature, such as from 85 to 205 °C. Preferably, the mixture is heated to a temperature of 90 to 160 °C, more preferably 95 to 130 °C, and yet more preferably 100 to 130 °C. The reaction may be carried out under oxygen-containing atmosphere, but an inert atmosphere is preferred. Following the reaction, the resulting polyarylene polymer may be isolated from the reaction mixture, diluted with appropriate solvent, or used as is for coating a surface. When a first monomer having 2 alkynyl moieties having terminal hydrogens and 1 alkynyl moiety having a terminal phenyl group is used to prepare the present polymers, heating the monomer reaction mixture at a temperature of 90 to 130 °C will provide an oligomer where substantially only the alkynyl moieties having terminal hydrogens react with the first monomer to form a linear oligomer having 1 or 2 third monomers as end caps, that is, the alkynyl moieties having the terminal phenyl group remain substantially unreacted (< 10%, and preferably < 5%, of such groups react).

The present polyarylene polymers may have any suitable molecular weight range, such as a weight average molecular weight (M_{w}) of from 500 to 250000 Da (as determined by gel permeation chromatography against polystyrene standards), preferably from 1000 to 100000 Da, and more preferably from 2000 to 50000 Da. The choice of organic solvent can be used to tailor the M_{w} of the resulting oligomer. For example, when aromatic ether solvents, such as C₁₋₆-alkoxy-substituted benzenes, are used, relatively higher M_{w} polymers may be obtained as compared to polymers having a relatively lower M_{w} when the same reaction is performed using a benzyl ester of a C₂₋₆-alkanecarboxylic acid as the organic solvent. The molecular weight of the present polymers can also be controlled, even in aromatic ether solvents, by adjusting the amount of the first monomer and/or optional monomers. For example, to obtain a polymer having a M_{w} of ≤ 35000, ≥ 1.05 mole of the first monomer should be used for each 1 mole of the second monomer, that is, the mole ratio of total polyalkynyl-substituted aryl monomers to total biscyclopentadienone second monomers should be ≥ 1:1.05, such as from 1:1.075 to 1:1.95. As the optional end-capping monomer has a single alkynyl moiety, it can be used to control the growth of the polymer chain. Increasing the total amount of any end-capping monomer in the reaction will generally provide polymers having relatively lower M_{w}, while decreasing the total amount of any end-capping monomer will provide resins having relatively higher M_{w}.

While not intending to be bound by theory, it is believed that the present polyarylene polymers are formed through the Diels-Alder reaction of the cyclopentadienone moieties of the second monomer with the alkynyl moieties of the first monomer and the alkynyl moieties of any optional end-capping monomers upon heating. During such Diels-Alder reaction, a carbonyl-bridged species is believed to form. It will be appreciated by those skilled in the art that such carbonyl-bridged species may be present in the oligomers. Upon further heating, it is believed that the carbonyl bridging species will be essentially fully converted to an aromatic ring system. Due to the mole ratio of the monomers used, the present polymers contain arylene rings in the polymer backbone which are substituted with at least one epoxy-reactive moiety as illustrated in the following reaction Scheme 1, where A is the first monomer and B is the second monomer, wherein polyalkynyl-substituted aryl first monomer A has an epoxy-reactive moiety Z.

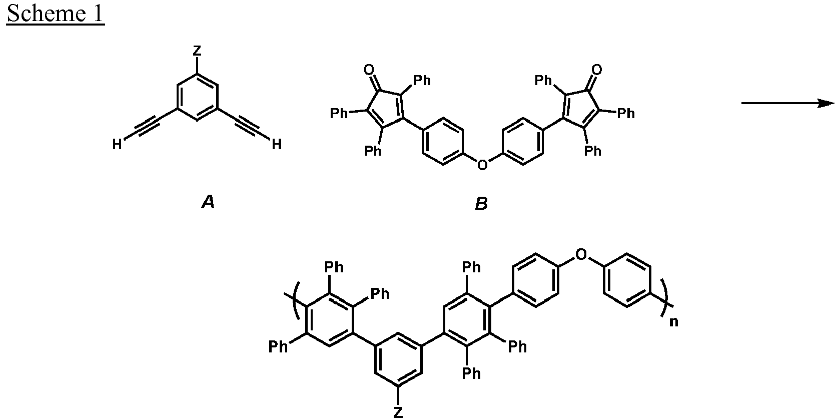

When end-capping monomers are not used, the present polyarylene resins have backbone termini independently chosen from alkynyl moieties and cyclopentadienyl moieties. If an excess of the first monomer is used, the polyarylene resin backbone will terminate in alkynyl moieties. If an excess of the second monomer is used, the polyarylene resin backbone will terminate in cyclopentadienyl moieties. Preferred polyarylene polymers are those having repeating units of the formula (16) 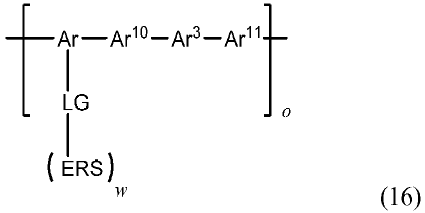 wherein LG, ERS, and *w* are as described above in formula (2); Ar is an optionally substituted C₅₋₃₀ aryl moiety; Ar¹⁰ and Ar¹¹ are each independently optionally substituted C₆₋₁₀ aryl moieties; Ar³ is as defined above for formula (11); and *o* is the number of repeat units in the oligomer and is an integer from 2 to 1000.

The present compositions comprise one or more epoxide-containing crosslinkers. As used herein, the term "epoxide-containing crosslinkers" refers to any epoxide-containing compound having 2 or more epoxide moieties capable of reacting with the epoxy-reactive moieties of the present polyarylene polymers to form a crosslinked polymer. Preferably, the epoxide-containing crosslinkers have from 2 to 6 epoxide moieties, more preferably from 2 to 4 epoxide moieties, and yet more preferably from 2 to 3 epoxide moieties. Exemplary epoxide moieties include, but are not limited to, glycidyl ether moieties and cyclohexene oxide moieties. A wide variety of epoxide-containing crosslinkers may be used in the present compositions. Suitable epoxide-containing crosslinkers include, without limitation: diglycidyl ethers of bisphenols such as bisphenol A diglycidyl ether, bisphenol E diglycidyl ether, bisphenol F diglycidyl ether, bisphenol S diglycidyl ether, bisphenol BP diglycidyl ether, bisphenol AF diglycidyl ether, bisphenol AP diglycidyl ether, and oligomeric bisphenol diglycidyl ethers; glycidyl ethers of polyols, such as 1,4-butanediol diglycidyl ether, 2-methyl-1,3-propanediol diglycidyl ether, 2,2-dimethyl-1,3-propanediol diglycidyl ether, glycerol triglycidyl ether, 1,4-cyclohexanedimethanol diglycidyl ether, trimethylolpropane triglycidyl ether, triphenylolmethane triglycidyl ether, bis(naphthyldiol)methane tetraglycidyl ether, resorcinol diglycidyl ether, 4,4'-biphenol diglycidyl ether, and trihydroxybenzene triglycidyl ether; glycidyl esters of polycarbocylic acids such as diglycidyl benzenedicarboxylate, triglycidyl benzenetricarboxylate, diglycidyl decanedicarboxylate, diglycidyl dodecanedicarboxylate, diglycidyl hexadecanedicarboxylate, diglycidyl octadecanedicarboxylate, diglycidyl eicosanedicarboxylate, and diglycidyl hexatridecanedicarboxylate; epoxidized natural oils such as castor oil triglycidyl ether; glycidyl ethers of polyether polyols such as triglycidyl ether of propoxylated glycerol, triglycidyl ether of ethoxylated glycerol, and triglycidyl of ethoxylated-propoxylated glycerol; epoxidized polyarylene polymers; epoxidized (meth)acrylate polymers and copolymers such as those comprising as polymerized units glycidyl acrylate and/or glycidyl methacrylate; and epoxidized siloxanes such as partially condensed silsesquioxanes having a plurality of cyclohexene oxide moieties, and polyoctahedral sislsesquioxanes having a plurality of cyclohexene oxide moieties. Suitable silicon-containing monomers useful in preparing epoxidized siloxanes have the structure 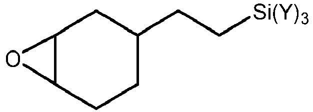 where Y is H, halogen, C₁₋₆ alkoxy, or C₁₋₆ carboxylate. Suitable epoxide-containing crosslinkers are generally commercially available or may be prepared by methods known in the literature.

The present compositions comprise one or more of the present polyarylene polymers, one or more epoxide-containing crosslinkers, and optionally one or more organic solvents. Preferably, the present compositions are free of polyester resins. Preferably, the compositions comprise one or more organic solvents. Any solvent which dissolves the polyarylene polymer and epoxide-containing crosslinker may suitably be used in the present composition. Exemplary organic solvents are those typically used in the electronics industry, such as propylene glycol methyl ether (PGME), propylene glycol methyl ether acetate (PGMEA), methyl 3-methoxypropionate (MMP), ethyl lactate, n-butyl acetate, anisole, N-methyl pyrrolidone, gamma-butyrolactone (GBL), ethoxybenzene, benzyl propionate, benzyl benzoate, propylene carbonate, xylene, cumene, limonene, mesitylene, and mixtures thereof. Mixtures of organic solvents are particularly preferred, such as a mixture comprising one or more of anisole, ethoxybenzene, PGME, PGMEA, GBL, MMP, n-butyl acetate, benzyl propionate and benzyl benzoate in combination with one or more additional organic solvents, and more preferably a mixture comprising two or more of anisole, ethoxybenzene, PGME, PGMEA, GBL, MMP, n-butyl acetate, benzyl propionate, and benzyl benzoate. When a mixture of solvents is used, the ratio of solvents is generally not critical and may vary from 99:1 to 1:99 w/w.

Any amount of the present polyarylene polymers may be used in the compositions of the invention. The epoxide-containing crosslinkers are typically used in an amount of from 1 to 50 wt% based on the weight of the polyarylene polymer, preferably from 3 to 40 wt%, and more preferably from 5 to 35 wt%. When the compositions of the invention comprise one or more organic solvents, the polyarylene polymers are typically present in an amount of 0.5 to 40 wt%. It will be appreciated by those skilled in the art that the concentration of the polyarylene polymer in the composition may be varied over a wide range depending on the particular application and coating method. The determination of suitable concentrations is within the ability of those skilled in the art.

The present compositions may further optionally contain one or more additives, such as curing agents and surface leveling agents. The selection of such optional additives and their amounts are well within the ability of those skilled in the art. Curing agents are typically present in an amount of from 0 to 20 wt% based on total solids, and preferably from 0 to 3 wt%. Surface leveling agents are typically used in an amount of from 0 to 5 wt% based on total solids, and preferably from 0 to 1 wt%.

Curing agents may optionally be used in the present compositions to aid in the curing of the deposited polymer film. A curing agent is any component which causes curing of the polymer on the surface of a substrate. Preferred curing agents are acids and thermal acid generators. Suitable acids include, but are not limited to: arylsulfonic acids such as p-toluenesulfonic acid; alkyl sulfonic acids such as methanesulfonic acid, ethanesulfonic acid, and propanesulfonic acid; perfluoroalkylsulfonic acids such as trifluoromethanesulfonic acid; and perfluoroarylsulfonic acids. A thermal acid generator is any compound which liberates acid upon exposure to heat. Thermal acid generators are well-known in the art and are generally commercially available, such as from King Industries, Norwalk, Connecticut. Exemplary thermal acid generators include, without limitation, amine blocked strong acids, such as amine blocked sulfonic acids such as amine blocked dodecylbenzenesulfonic acid. It will also be appreciated by those skilled in the art that certain photoacid generators are able to liberate acid upon heating and may function as thermal acid generators.

The present compositions may optionally include one or more surface leveling agents (or surfactants). While any suitable surfactant may be used, such surfactants are typically non-ionic. Exemplary non-ionic surfactants are those containing an alkyleneoxy linkage, such as ethyleneoxy, propyleneoxy, or a combination of ethyleneoxy and propyleneoxy linkages.

Compositions of the invention may be used to form a crosslinked polymer film in a variety of applications. For example, the present compositions may be coated on an electronic device substrate by any suitable means, such as spin-coating, slot-die coating, doctor blading, bar coating, curtain coating, roller coating, spray coating, dip coating, and the like. Spin-coating is preferred. In a typical spin-coating method, the present compositions are applied to a substrate which is spinning at a rate of 500 to 4000 rpm for a period of 15 to 90 seconds to obtain a desired layer of the composition on the substrate. It will be appreciated by those skilled in the art that the height of the layer may be adjusted by changing the spin speed.

A wide variety of electronic device substrates may be used in the present invention, such as: packaging substrates such as multichip modules; flat panel display substrates such as flat panel display substrates and flexible display substrates; integrated circuit substrates; substrates for light emitting diodes (LEDs) including organic light emitting diodes (OLEDs); semiconductor wafers; polycrystalline silicon substrates; and the like. Such substrates are typically composed of one or more of silicon, polysilicon, silicon oxide, silicon nitride, silicon oxynitride, silicon germanium, gallium arsenide, aluminum, sapphire, tungsten, titanium, titanium-tungsten, nickel, copper, and gold. Suitable substrates may be in the form of wafers such as those used in the manufacture of integrated circuits, optical sensors, flat panel displays, integrated optical circuits, and LEDs. As used herein, the term "semiconductor wafer" is intended to encompass "an electronic device substrate," "a semiconductor substrate," "a semiconductor device," and various packages for various levels of interconnection, including a single-chip wafer, multiple-chip wafer, packages for various levels, or other assemblies requiring solder connections. Such substrates may be any suitable size, such as wafers having a diameter of 200 mm to 300 mm. As used herein, the term "semiconductor substrate" includes any substrate having one or more semiconductor layers or structures which include active or operable portions of semiconductor devices. A semiconductor device refers to a semiconductor substrate upon which at least one microelectronic device has been or is being batch fabricated. Preferred substrates are display substrates and semiconductor substrates.

Optionally, a layer of an adhesion promoter may be applied to the substrate surface before the deposition of the polyarylene resin layer, which is subsequently cured to form a crosslinked polyarylene film. If it is desired to use an adhesion promoter, any suitable adhesion promoter for polyarylene films may be used, such as silanes, preferably organosilanes such as trimethoxyvinylsilane, triethoxyvinylsilane, hexamethyldisilazane [(CH₃)₃Si-NH-Si(CH₃)₃], or an aminosilane coupler such as gammaaminopropyltriethoxysilane, or a chelate such as aluminum monoethylacetoacetatedi-isopropylate [((i-C₃H₇O)₂Al(OCOC₂H₅CHCOCH₃))]. In some cases, the adhesion promoter is applied from 0.01 to 5 wt% solution, excess solution is removed, and then the polyarylene oligomer is applied. In other cases, for example, a chelate of aluminum monoethylacetoacetatedi-isopropylate, can be incorporated onto a substrate by spreading a toluene solution of the chelate on a substrate and then baking the coated substrate at 350 °C for 30 min. in air to form a very thin (for example 5 nm) adhesion promoting layer of aluminum oxide on the surface. Other means for depositing aluminum oxide are likewise suitable. Alternatively, the adhesion promoter, in an amount of, for example, from 0.05 to 5 wt% based on the weight of the polyarylene resin, can be added to the present compositions, negating the need for formation of an additional layer. Particularly suitable adhesion promoters include those sold under the AP 3000, AP 8000, and AP 9000S designations, available from Dow Electronic Materials (Marlborough, Massachusetts).

After being coated on the substrate, the polyarylene resin layer is optionally baked at a relatively low temperature to remove any organic solvent and other relatively volatile components from the layer. Typically, the substrate is baked at a temperature of 90 to 140 °C, although other suitable temperatures may be used. The baking time is typically from 10 seconds to 10 minutes, and preferably from 30 seconds to 5 minutes, although longer or shorter times may be used. When the substrate is a wafer, such baking step may be performed by heating the wafer on a hot plate. Following solvent removal, a layer, film or coating of the polyarylene resin and epoxide-containing crosslinker on the substrate surface is obtained.

Next, the layer of the polyarylene resin and epoxide-containing crosslinker is cured to form a crosslinked polyarylene film. Any suitable curing step may be employed, such as heating. Preferably, the layer is cured by heating at a temperature of ≥150 °C, more preferably at a temperature of 150 to 250 °C, and more preferably 160 to 225 °C. An advantage of the present compositions is that they can be cured at relatively lower temperatures as compared to conventional polyarylene resins that do not have one or more epoxy-reactive moieties. The cured, that is crosslinked, polyarylene films may be used as is, or may be further processed as needed, depending on the application. Such further processing steps are conventional and include, for example, the steps of etching and metalizing, and are well known to those skilled in the art.

In the following examples, weight average molecular weight (M_{w}) was determined by gel permeation chromatography (GPC) against polystyrene standards, and number average molecular weight (Mₙ) was determined by GPC using a dynamic light scattering/refractive index (DLS/RI) sensor.

Example 1: Preparation of Polymer 1. To a multineck round-bottomed flask containing a stir bar, diphenylene oxide bis(triphenylcyclopentadienone) (DPO-CPD, 15.00 g, 19.16 mmol), 3,5-diethynylbenzoic acid (DEBzOH, 1.793 g, 10.54 mmol) and 1,3,5-tris(phenylethynyl)benzene (TRIS, 3.988 g, 10.54 mmol) were added via powder funnel, followed by GBL (48 g) as the reaction solvent. The reaction was stirred gently at room temperature. The flask was next equipped with a reflux condenser and an internal thermocouple probe attached to a self-regulating thermostat control for a heating mantle. Next, the dark maroon contents of the flask were warmed to an internal temperature of 203 °C and maintained at this temperature for 60 hours before cooling to 25 °C by removal of the heating element. The resulting maroon solution was precipitated from GBL using 300 mL water heated to 70 °C as an antisolvent. Filtration and drying of the precipitate in a vacuum oven for 3 days yielded Polymer 1 as an off-white powder. Polymer 1 was analyzed by GPC to provide an Mₙ of 6761 Da, an M_{w} of 41719 Da, and a polydispersity index (M_{w}/Mₙ) of 6.17. This reaction is shown in Scheme 2.

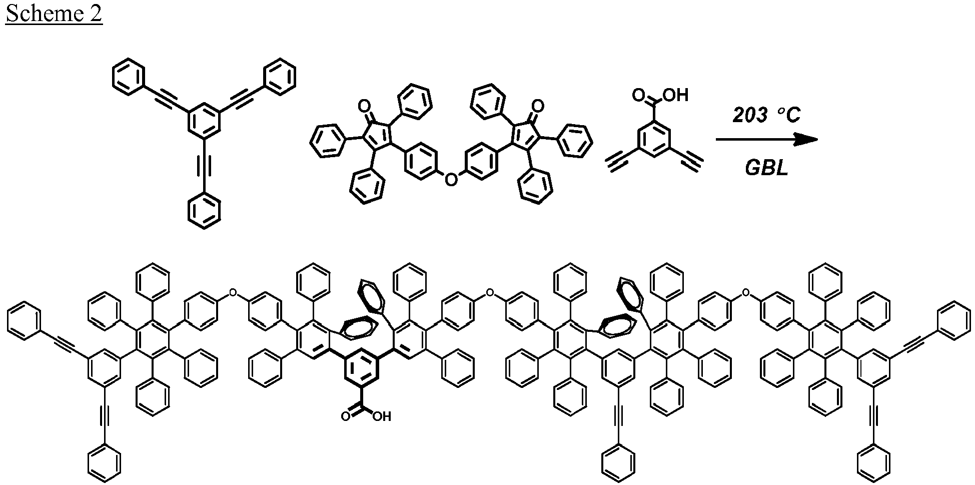

Example 2: Preparation of Polymer 2. DPO-CPD (9.0 g, 0.0115 mol) and 3,5-diethynylphenol (1.96 g, 0.0138 mol) were dissolved in 97 g of GBL. The reaction was heated at 120 °C for 1 hr. and then 130 °C for 1 hr. and then 150 °C for 1.5 hr. The mixture was cooled to room temperature and then diluted with 10 g of GBL. The reaction mixture was slowly added to warm water. The precipitated polymer (Polymer 2) was collected by filtration and then dried in vacuum oven at 65 to 70 °C for 2 days, yielding 10.0 g of a brown solid in 97% yield. Analysis of the polymer by GPC indicated an M_{w} of 9300, and a polydispersity index (M_{w}/Mₙ) of 2.1. This reaction is illustrated in Scheme 3.

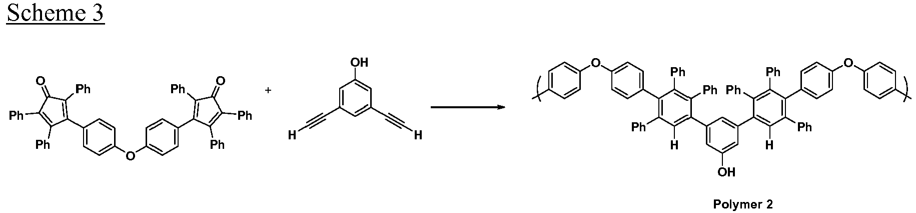

Example 3: Preparation of Polymer 3. DPO-CPD (9.0 g, 0.0115 mol) and 3,5-diethynylphenol (1.37 g, 0.0096 mol) were dissolved in 97 g of GBL. The reaction was heated at 130 °C for 2 hr. and then cooled down to 80 °C. TRIS (1.57 g, 0.0041 mol) was then added to reaction mixture at 80 °C. The reaction was then heated at 190 °C for 16 hr. The reaction mixture was cooled to room temperature and then diluted with GBL (10 g). The reaction mixture was slowly added to warm water. The precipitated polymer was collected by filtration and then dried in vacuum oven at 65 to 70 °C for 2 days. Polymer 3 was obtained as a brown solid (10.1 g) in 98% yield. Analysis by GPC indicated a M_{w} of 7100 and a polydispersity index of 3.7.

Example 4: Preparation of Polymer 4. The procedure of Example 1 was generally repeated except that no TRIS monomer was used. The resulting polymer, Polymer 4, had an Mₙ of 20.7 kDa.

Example 5: Polymers 5-9. The procedure of Example 1 is repeated except that 3,5-diethynylbenzoic acid is replaced with the monomers shown in Table 1 with similar results expected.

**Table 1**

| **Polymer Number** | **Monomer** |
|---|---|
| 5 | 2-(3,5-Diethynylphenoxy)ethan-1-ol |
| 6 | 3,5-Diethynylaniline |
| 7 | 3,5-Bis(phenylethynyl)benzoic acid |
| 8 | 6,6'-Diethynyl-[1,1'-binaphthalene]-2,2'-diol |
| 9 | 4,9-Diethynylpyrene-1,6-dicarboxylic acid |

Comparative Polymers. Comparative Polymer 1 was prepared according to the general procedure of Example 1 except that no DEBzOH monomer was used. The resulting polymer, Comparative Polymer 1, was precipitated from water and was found to have an Mₙ of 9.7 kDa.

Comparative Polymer 2 was prepared according to the general procedure of Example 4 except that the DEBzOH monomer was replaced with 3,5-diethynylbenzamide. The ratio of 3,5-diethynylbenzamide to DPO-CPD was 1.1:1.

Example 6. Bisphenol A diglycidyl ether (BPA-DGE, 20 mg) was added to 1 g of a 10 wt% solution in PGMEA of a polyarylene oligomer in a 20 mL scintillation vial. The polyarylene oligomers used are shown in Table 2. The Control sample was BPA-DGE alone in PGMEA with no polyarylene oligomer present.

**Table 2**

| **Formulation No.** | **Polyarylene Oligomer** |
|---|---|
| 1 | Polymer 4 |
| C1 | Comparative Polymer 1 |
| C2 | Comparative Polymer 2 |
| Control | None |

Each vial was warmed in a heating block to 100 °C without a cap, allowing for the evaporation of solvent and the formation of a thick film on the bottom of the vial. The temperature of the heating block was raised to 165 °C, and the vial was kept at this temperature for 2 hrs. Each resulting film was dissolved in tetrahydrofuran (THF), and diluted for analysis by GPC. GPC analysis of the films formed from each of Formulations C1 and C2 indicated little to no reaction occurred. GPC analysis of the film formed from Formulation 1 showed significant broadening of the molecular weight distribution with a concomitant increase in polydispersity, indicating significant crosslinking occurred. GPC analysis of the control sample showed no change.

Example 7. Formulation 2 was prepared according to the procedure of Example 6 using Polymer 4 and BPA-DGE, except that the amount of BPA-DGE was 50 wt%, based on the weight of Polymer 4. Following the procedure of Example 6, GPC analysis of the THF extract of the film formed from Formulation 2 indicated complete crosslinking.

Example 8. Various formulations of the invention are expected to be prepared by combining the epoxide-containing crosslinkers and polyarylene oligomers shown in Table 3 in PGMEA. The amount of the crosslinker reported in Table 3 is in wt%, based on the weight of the polyarylene oligomer. The following abbreviations are used in Table 3: BPF-DGE = bisphenol F diglycidyl ether; BPBP-DGE = bisphenol BP diglycidyl ether; TPM-DGE= triphenylolmethane triglycidyl ether; CO-TGE = castor oil triglycidyl ether; PG-TGE = triglycidyl ether of propoxylated glycerol having on average 8 moles of propoxylation; and G-TGE = glycerol triglycidyl ether; BNM-TTGE = bis(naphthyldiol)methane tetraglycidyl ether.

**Table 3**

| **Formulation No.** | **Crosslinker** | **Polyarylene Oligomer** |
|---|---|---|
| 3 | BPF-DGE (15 wt%) | Polymer 1 |
| 4 | BPBP-DGE (25 wt%) | Polymer 4 |
| 5 | TPM-TGE (15 wt%) | Polymer 2 |
| 6 | PG-TGE (20 wt%) | Polymer 2 |
| 7 | G-TGE (30 wt%) | Polymer 9 |
| 8 | TPM-TGE (25 wt%) | Polymer 7 |
| 9 | CO-TGE (23 wt%) | Polymer 6 |
| 10 | BPA-DGE (35 wt%) | Polymer 6 |
| 11 | BNM-TTGE (30 wt%) | Polymer 8 |
| 12 | BPA-DGE (30 wt%) | Polymer 5 |

## Claims

1. A composition comprising: one or more polyarylene polymers having a backbone comprising as repeating units one or more aryl moieties having one or more epoxy-reactive moieties, the polyarylene polymer comprising as polymerized units one or more polyalkynyl-substituted aryl first monomers and one or more biscyclopentadienone second monomers; and one or more epoxide-containing crosslinkers.

2. The composition of claim 1 wherein the epoxy-reactive moiety has the formula 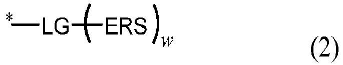 where LG is a linking group or a single chemical bond; each ERS is an epoxy-reactive substituent; w is an integer from 1 to 6; and * is the point of attachment to an aryl moiety.

3. The composition of claim 2 wherein each epoxy-reactive substituent is independently chosen from -C(=O)-OH, -OH, -SH, -NR²⁰R²¹, and combinations thereof, wherein R²⁰ and R²¹ are independently chosen from H, C₁₋₁₀-alkyl, C₆₋₁₀-aryl, and C₇₋₂₀-aralkyl.

4. The composition of claim 1 wherein the one or more first monomers comprise an aryl moiety substituted with the one or more epoxy-reactive moieties.

5. The composition of claim 4 wherein the one or more second monomers are chosen from compounds of the formula 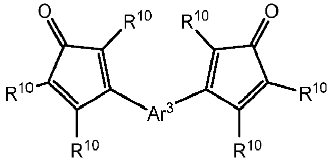 wherein each R¹⁰ is independently chosen from H, phenyl, or substituted phenyl; and Ar³ is an aryl moiety.

6. The composition of claim 1 wherein at least one polyarylene polymer has repeating units of the formula 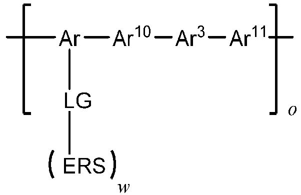 wherein LG is a linking group or a single chemical bond; each ERS is an epoxy-reactive substituent; *w* is an integer from 1 to 6; Ar is an optionally substituted C₅₋₃₀ aryl moiety; Ar¹⁰ and Ar¹¹ are each independently optionally substituted C₆₋₁₀ aryl moieties; Ar³ is an aryl moiety; and *o* is the number of repeat units in the oligomer and is an integer from 2 to 1000.

7. The composition of claim 1 further comprising one or more organic solvents.

8. A method comprising:
providing a substrate;
coating a layer of the composition of claim 7 on a surface of the substrate; and
curing the layer of the composition to form a cross-linked polyarylene layer.

9. A composition comprising: (a) one or more polyarylene polymers, at least one polyarylene polymer comprising as polymerized units one or more monomers of the formula 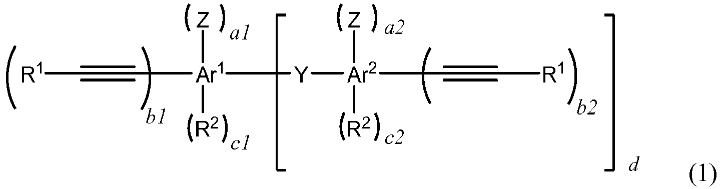 wherein Ar¹ and each Ar² are independently a C₅₋₃₀-aryl moiety; each R¹ is independently chosen from H, C₅₋₃₀-aryl, and substituted C₅₋₃₀ aryl; each R² is independently chosen from C₁₋₁₀-alkyl, C₁₋₁₀-haloalkyl, C₁₋₁₀-alkoxy, CN, and halo; each Z is an epoxy-reactive moiety; Y is a chemical bond or a divalent linking group chosen from -O-, -S-, -S(=O)-, -S(=O)₂-, - C(=O)-, -(C(R⁵)₂)*_{z}*-, C₅₋₃₀-aryl, and -(C(R⁵)₂)*_{z1}*-(C₅₋₃₀-aryl)-(C(R⁵)₂)*_{z2}*-; each R⁵ is independently chosen from H, hydroxy, halo, C₁₋₁₀-alkyl, C₁₋₁₀-haloalkyl, and C₅₋₃₀-aryl; *a1* = 0 to 3; each *a2* = 0 to 3; *b1* = 1 to 4; each *b2* = 0 to 2; *c1* = 0 to 2; each *c2* = 0 to 2; *a1* + each *a2 =* 1 to 6; *b1* + each *b2* = 2 to 6; *c1* + each *c2* = 0 to 6; *d* = 0 to 2; *z* = 1 to 10; *z1* = 0 to 10; *z2* = 0 to 10; and *z1* + *z2* = 1 to 10; and (b) one or more epoxide-containing crosslinkers.

10. The composition of claim 9 wherein each Z is independently has the formula 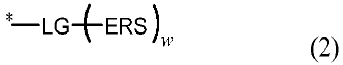 where LG is a linking group or a single chemical bond; each ERS is an epoxy-reactive substituent; *w* is an integer from 1 to 6; and * is the point of attachment to an aryl moiety.

11. The composition of claim 10 wherein the epoxy-reactive substituent is chosen from one or more of -C(=O)-OH, -OH, -SH, -NR²⁰R²¹, and combinations thereof, wherein R²⁰ and R²¹ are independently chosen from H, C₁₋₁₀-alkyl, C₆₋₁₀-aryl, and C₇₋₂₀-aralkyl.

12. The composition of claim 11 wherein the one or more polyarylene polymers further comprise as polymerized units one or more second monomers comprising two cyclopentadienone moieties.

13. The composition of claim 12 wherein the one or more second monomers are chosen from one or more monomers of formula 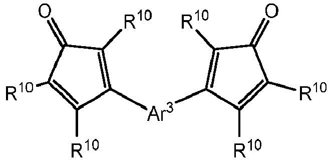 wherein each R¹⁰ is independently chosen from H, phenyl, or substituted phenyl; and Ar³ is an aryl moiety.

14. The composition of claim 9 further comprising one or more organic solvents.

15. A method comprising:
providing a substrate;
coating a layer of the composition of claim 14 on a surface of the substrate; and
curing the layer of the composition to form a cross-linked polyarylene layer.
